# EUROPEAN PATENT APPLICATION

(11) **EP 0 953 352 A1**
(43) Date of publication of application: **03.11.1999**
(21) Application number: 99107436.0
(22) Date of filing: 27.04.1999
(51) Int. Cl.: A61K 35/36

(54) **A therapeutic agent for ischemic diseases**

(30) Priority: 27.04.1998 JP 13461998
(71) Applicant: NIPPON ZOKI PHARMACEUTICAL CO., LTD., Chuo-ku, Osaka (JP)
(72) Inventor: Konishi, Jin-emon, Musashino-shi, Tokyo (JP); Fukuhara, Koki, 442-1 Kinashi, Yashiro-cho, Katoh, Hyogo (JP)
(74) Representative: HOFFMANN - EITLE

(57) **Abstract**

An object of the present invention is to offer a therapeutic agent for ischemic diseases having little side effects and being capable of administering safely.

The effective component of the therapeutic agent for ischemic diseases of the present invention is an extract from inflammatory tissue inoculated with vaccinia virus.

An extract from inflammatory tissue inoculated with vaccinia virus. protects a cell against damage by suppressing abnormal depolarization of hyppocampal pyramidal neurons during hypoxia and so can delay the time falling into cell death. The characteristic action is not observed for normal pyramidal neurons, but is expressed only in the stage of pathema during hypoxia. Therefore, the substance of the present invention is useful as a drug to treat and prevent ischemic diseases such as cerebral infarction and the like and symptoms accompanying with neuropathy caused by ischemia.

## Description

### Detailed Description of the Invention:

### [Technical Field]

The present invention relates to a novel pharmacological action of an extract from inflammatory tissue inoculated with vaccinia virus. More particularly, it relates to a therapeutic agent for ischemic diseases containing the extract from inflammatory tissue inoculated with vaccinia virus as an effective component.

### [Prior Art]

An ischemic condition of brain causes a damage of nerve cells and lead them to necrosis, because supplies of oxygen and energy to brain are cut off. As the result, each of cells cannot maintain its homeostasis. Cerebral infarction is a diseased state that cerebral tissues irreversibly fall into necrosis by ischemia, and cerebral thrombosis or cerebral embolism is given given as a cause of it. Cerebral thrombosis is an arteria obliteration accompanying with a formation of thrombus induced by hemadostenosis. The hemadostenosis is caused by a progression of arteriosclerotic lesion in cerebral arteria. Cerebral embolism is induced from cerebral arteria embolism by thrombus. The thrombus on the wall of heart caused by cardic deseases or relating atheromatous degeneration in aorta and/or carotid arteries is separated and carried to the cerebral lesion, and results in causing cerebral arteria embolism.

Up to now, it is well realized clinically that neurocytes are very weak against ischemia. Some kinds of neurocytes are damaged by just a few minutes ischemia and lead to cell death. The hypoxia model is supposed as the condition of acute phase ischemia in clinical and is used as a model of cerebral apoplexy by using cerebral neurocytes. In ischemic hippocampal pyramidal neurons, a block of nerve conduction was occurred after a remarkable excite of the nerve accompanying with depolarization. After that, it is believed that the cells are damaged by cytotoxicity accompanying with increasing of glutamic acid in outer cells, calcium ion in the cells and free radicals, etc., and as a result, the damaged cells lose the function and lead to cell death. Therefore, a substance suppressing the depolarization of neurocytes in the course of cell damage during ischemia is useful as a drug for treatment and prevention of ischemic diseases.

### [Problems to be Solved by the Invention]

The present inventors have conducted various tests and studies on pharmacological activity of an extract from inflammatory tissue inoculated with vaccinia virus and, as a result, they have found that said substance has a novel action of suppressing abnormal depolarization of neurocytes during ischemia. This novel pharmacological action of the substance of the present invention has an excellent characteristic, that is, the action is not observed in normal condition, but is expressed only in the stage of pathema during hypoxia

### [Means to Solve the Problems]

An object of the present invention is to offer a therapeutic agent for ischemic diseases having little side effects and being capable of administering safely because of expressing its pharmacological effects only in the stage of pathema.

### [Embodiments of the Invention]

It has been known that, against invasion from outside by virus, etc. and against progress of inner diseases state, living body produces various biofunction-regulating substances for maintaining its homeostasis and for regulating and normalizing the biofunctions by means of two phases consisting of a suppressing action to excessive reactions and an enhancing action to depression of functions. For example, there have been various reports on biofunction-regulating substances which are produced in inflammatory tissue inoculated with vaccinia virus, methods for extracting said substances from diseased tissues and pharmacological activities thereof (refer, for example, to the Japanese Examined Patent Publications Sho-63/039,572 B, Sho-63/025,600 B, Hei-03/043,279 B and Japanese Patent 2,594,222).

As to a drug which is actually available, there is a drug preparation of an extract from inflammatory rabbit skin inoculated with vaccinia virus. As mentioned in page 1,434 of "Drugs in Japan, Ethical Drugs" (published in August of 1994; edited by Japan Pharmaceutical Information Center; published by Yakugyo Jiho Co., Ltd.), this preparation is a drug containing a non-protein active substances extracted and isolated from inflammatory tissues of rabbits inoculated with vaccinia virus, has been allowed to use for low back pain, neck-shoulder-arm syndromes, periarthritis scapulohumeralis, osteoarthritis, symptomatic neuralgia, itching accompanied with skin disorders (such as eczema, dermatitis and urticaria), allergic rhinitis, sequelae of subacute myelo-optico-neuropathy (such as coldness, pain and paresthesia/dysesthesia), etc., which is approved as an ethical drug in the forms of injections (subcutaneous, intramuscular and intravenous) and of tablets and commercially available.

The effective component of the therapeutic agent for ischemic diseases of the present invention is a non-protein biofunction-regulating substance extracted from inflammatory tissues inoculated with vaccinia virus. A drug preparation of an extract from inflammatory rabbit skin inoculated with vaccinia virus which is listed in the above-mentioned "Drugs in Japan, Ethical Drugs" has been approved as a pharmaceutical agent, put into the market and available in Japan. In addition, various extracts from inflammatory tissue inoculated with vaccinia virus mentioned in the references such as the above-mentioned patent publications can be utilized as the active substance of the present invention and their manufacturing methods and preferred doses are illustrated in the references as well.

With regard to the route of administration to the patient, subcutaneous, intramuscular and intravenous administrations by injection and oral administration by tablets are approved in the commercially available agent but it is also possible to administer the pharmaceutical dosage forms other than the above-mentioned ones which are optimum for the therapy depending upon the type of the disease. The dose is to be suitably decided depending upon the kind of an extract from inflammatory tissue inoculated with vaccinia virus while the dose which is approved in the commercially available preparation according to the above "Drugs in Japan, Ethical Drugs" (page 1,434) is, principally, 16 Neurotropin units per day and 3.6-7.2 Neurotropin units per day by oral administration and by injection, respectively. However, the dose may be appropriately increased or decreased depending upon the type of the disease, degree of seriousness, individual difference in the patients, method of administration, period of administration, etc.

As hereunder, results of the pharmacological tests concerning the novel pharmacological action of an extract from inflammatory tissue inoculated with vaccinia virus are given.

### [Examples]

### (1) Methods for electrophysiological study on hippocampal pyramidal cells

Male Wistar rats weighing 140 to 200 g were used for the experiments. Brain was rapidly removed and kept for 15 min in ice-bath, with oxygenated (95 % O₂ + 5 % CO₂) artificial cerebrospinal fluid ,ACSF (124 mM NaCl, 5 mM KCl, 2.6 mM CaCl₂, 1.3 mM MgSO₄, 1.24 mM KH₂PO₄, 26 mM NaHCO₃, 10 mM Glucose, pH 7.4). Hippocampus was dissected out, and transversal slices (400 µm thick) were cut with a micro-slicer and incubated with oxygenated ACSF kept at room temperature for over 30 min. The slices were transferred to the recording chamber, and perfused with oxygenated ACSF at 34.0±0.2 °C, a flow rate of 1.5 mL/min. After stabilization for 30 min, intracellular recording of pyramidal cells in the CA1 region was performed using sharp glass electrodes (tip resistance 40 to 90 M Ω) filled with 4 M potassium acetate. The electrode was inserted along the plane of CA1 stratum pyramidale under a stereoscopic microscope. Resting membrane potentials were measured by using an intracellular recording amplifier. Input membrane resistance were monitored by injection of hyperpolarizing currents (current - 0.2 nA, duration 200 ms, interval 0.2 Hz). Pyramidal cells with stable membrane potentials more negative than - 55 mV were used for the experiment.

### (2) Effect of a tested drug under normoxic conditions

This experiment was performed under normoxic conditions. Effects of a tested drug on membrane potentials and resistance were evaluated with current-voltage (I/V) curves obtained before (5 min) and during (10 to 20 min) drug application. The I/V curves were calculated by measuring the membrane response to intracellular current pulses of varying amplitude (current -0.1 to -0.5 nA, duration 200 ms, interval 0.2 Hz). Statistical differences in membrane potentials and resistance between before and during drug application were analyzed using a paired t-test.

As a tested drug, the substance of the present invention (a preparation of an extract from inflammatory skin inoculated with vaccinia virus; trade name: Neurotropin) diluted with ACSF (0.03 to 0.3 NU/ml as final concentration) was bath-applied.

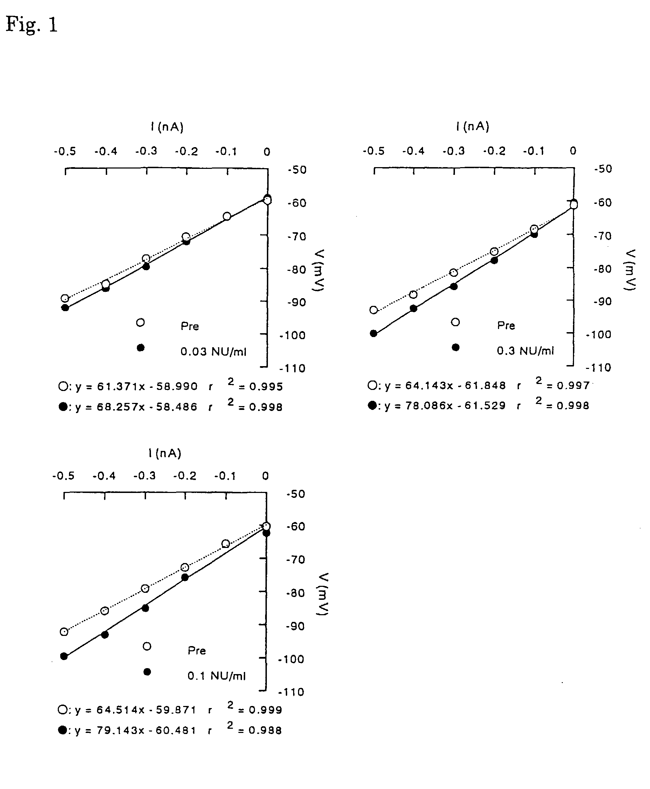

The effect of said tested drug on the I/V relationship of pyramidal cells was demonstrated in Fig. 1. Each point represents the mean value of 4 cells. Tested drug at the dose of 0.03 to 0.3 NU/mL had no significant effects on the membrane potential (the intersection on the Y axis). The membrane resistance (regression coefficient) showed the tendency to increase after the application of the tested drug at doses more than 0.1 NU/mL.

### (3) Effect of a tested drug under hypoxic conditions

Hypoxia (less than 60 mmHg PO₂ in the bathing medium) was produced by switching bathing medium to a 95% N₂ + 5% CO₂ bubbled ACSF and the same gas was administered over the surface of the chamber. A tested drug was pre-treated for 10 min under normoxic conditions, and then treated for 20 min during hypoxic conditions. Membrane potentials and resistance in drug-treated cells were measured throughout the period of normoxic and hypoxic period, and were compared with those of drug-untreated cell that was provided as control group. Statistical differences in membrane properties among control and drug-treated groups were analyzed using a one-way ANOVA.

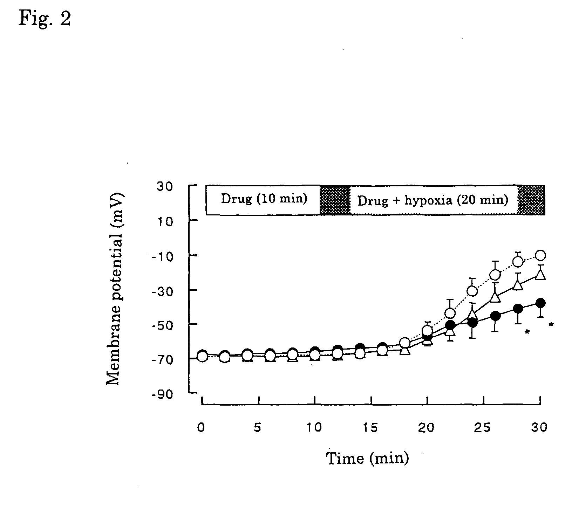

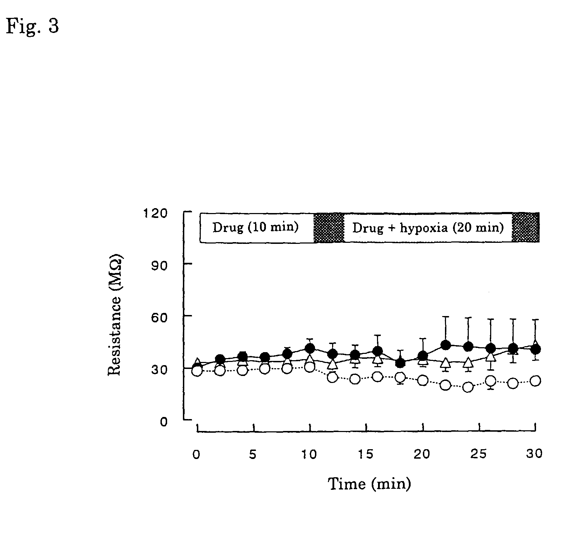

Effects of the substance of the present invention on changes in membrane potentials of pyramidal cells throughout the period of normoxic and hypoxic period were shown in Fig. 2. Data represents the mean± standard error of 5 to 6 cells. Basal resting potentials in each group were - 69.1±0.9 (control group), -67.9±1.5 (low dose-treated group) and -67.9 ±1.5 (high dose-treated group), respectively. Effects of the substance of the invention on changes in membrane resistance of pyramidal cells throughout the period of normoxic and hypoxic period were shown in Fig. 3. Data represents the mean±standard error of 4 to 5 cells. Basal input resistance in each group were 28.2 ±1.2 (control group), 30.3 ± 2.9 (low dose-treated group) and 33.1±2.0 MΩ (high dose-treated group), respectively.

In control group, a rapid depolarization began about 6 min after hypoxic exposure, and then reached a plateau levels of about - 10 mV within 20 min (Fig. 2). On the other hand, membrane input resistance decreased 1 or 2 min after hypoxic exposure and this reduction remained during hypoxia (Fig. 3). Successive treatment of 0.1 NU/mL of the substance of the present invention before and during hypoxia had a tendency to inhibit hypoxia-induced depolarization, and the treatment of 0.3 NU/mL of the present substance suppressed significantly hypoxia-induced depolarization (Fig. 2). Furthermore, the substance of the invention at both doses had a tendency to restore the reduction in resistance induced by hypoxia (Fig. 3). The substance of the present invention obtained by a manufacturing method mentioned in Example 1 of the Japanese Patent 2,594,222 was tested in the same manner. The substance also showed an inhibitory effect against hypoxia-induced depolarization dose-dependently as well as said preparation of an extract from inflammatory skin inoculated with vaccinia virus which is commercially available.

### [Merit of the Invention]

It is apparent from the results of the above-mentioned pharmacological tests that the substance of the present invention has an excellent action, i.e., the substance protects a cell against damage by suppressing abnormal depolarization of hyppocampal pyramidal neurons during hypoxia and so can delay the time falling into cell death. The characteristic action of the substance of the present invention is not observed for normal pyramidal neurons, but is expressed only in the stage of pathema during hypoxia.

As a mechanism of suppression against abnormal depolarization of nerve cells in the ischemic state, an activation of Na⁺-K⁺ pump, an suppression of Na⁺ or Ca²⁺ channel, and an activation of K⁺ or Cl⁻channel are suggested electrophysiologically. In the course of cell damage during ischemia, a drug suppressing depolarization of neurons can treat cerebral infarction which is directly caused by ischemia and prevent the worse of it. Also, such drug can improve and protect a disturbance of memory or consciousness in vascular dementia caused by hyppocampal disturbance during ischemia. Furthermore, in consideration of said mechanism of inhibitory action against abnormal depolarization, the drug has a possibility to improve cerebral and peripheral neuropathy caused by ischemia, in particular, symptoms accompanying with hypersthenia of motor and sensory nerve. Therefore, the substance of the present invention is very useful as a drug to treat and prevent various ischemic diseases.

### Brief Description of the Drawings:

[Fig. 1]
   Fig. 1 is a graph showing the effect of substance of the present invention on the current-voltage (I-V) relationship of the pyramidal neurons in hyppocampal CA1. In the figure, a white circle represents a result of a control group and a black circle represents that of a tested drug group. Doses of the tested drug (the substance of the present invention) is shown in the figure.
[Fig. 2]
   Fig. 2 is a graph showing the effect of substance of the present invention on resting membrane potential of pyramidal neurons of hyppocampal CA1 during hypoxia. In the figure, a white circle represents a result of a control group (only artificial cerebrospinal fluid), a white triangle represents that of a group treated with the substance of the present invention (0.1 NU/mL). and a black circle represents that of a group treated with the substance of the invention (0.3 NU/mL). The mark * indicates p<0.05 significance versus control group.
[Fig. 3]
   Fig. 3 is a graph showing the effect of substance of the present invention on membrane resistance of pyramidal neurons of hyppocampal CA1 during hypoxia. In the figure, a white, a white triangle and a black circle are the same representations as Fig. 2.

## Claims

1. Use of an extract from inflammatory tissue inoculated with vaccinia virus for the production of a pharmaceutical composition effective in the treatment of ischemic diseases.

2. Use according to claim 1, wherein the inflammatory tissue is a skin tissue.

3. Use according to claim 2, wherein the inflammatory tissue is a rabbit skin tissue.

4. Use according to any of the claims 1 to 3, wherein the composition is a composition for injection.

5. Use according to any of the claims 1 to 3, wherein the composition is a composition for oral administration.

6. Use according to any of the claims 1 to 5, wherein the ischemic disease is ischemic brain disease.
